Europäisches Patentamt

European Patent Office

Office européen des brevets

(19)

(11) Numéro de publication: **0 082 762
B1**

(12) **FASCICULE DE BREVET EUROPEEN**

(45) Date de publication du fascicule du brevet:
**29.05.85**

(51) Int. Cl.⁴: **C 07 C 101/72**

(21) Numéro de dépôt: **82402285.9**

(22) Date de dépôt: **14.12.82**

(54) **Diméthylamino-2 parahydroxyphényl-2 acétate de sodium, son procédé de préparation et son application à la fabrication du cyanure de parahydroxybenzyle.**

(30) Priorité: **23.12.81 FR 8124104**

(43) Date de publication de la demande:
**29.06.83 Bulletin 83/26**

(45) Mention de la délivrance du brevet:
**29.05.85 Bulletin 85/22**

(84) Etats contractants désignés:
**BE CH DE FR GB IT LI NL SE**

(56) Documents cités:
**DE - A - 2 118 988
FR - A - 2 131 803**

**CHEMICAL ABSTRACTS, vol. 84, no. 19, 10 mai 1976,
page 151, no. 131504z, Columbus, Ohio, USA**

(73) Titulaire: **SOCIETE FRANCAISE HOECHST Société
anonyme dite:, 3, avenue du Général de Gaulle,
F-92800 Puteaux (FR)**

(72) Inventeur: **Gallois, Philippe, 32 Square du Maine,
F-95470 Fosses (FR)**
Inventeur: **Christidis, Yani, 12, Rue de Constantinople,
F-75008 Paris (FR)**

(74) Mandataire: **Rinuy, Santarelli et al, 14, avenue de la
Grande Armée, F-75017 Paris (FR)**

BUNDESDRUCKEREI BERLIN

## Description

La présente invention concerne, à titre de produit industriel nouveau, le diméthylamino-2 parahydroxyphényl-2 acétate de sodium cristallisé anhydre ou avec deux molécules d'eau, son procédé d'obtention et son application à la fabrication du cyanure de parahydroxybenzyle.

Le diméthylamino-2 parahydroxyphényl-2 acétate de sodium cristallisé anhydre ou avec deux molécules d'eau n'est pas décrit dans la littérature. La demande de brevet N° 78.21174, publiée sous le N° 2.431.405, enseigne un procédé pour accéder à l'acide amino-2 parahydroxyphényl-2 acétique, ou l'un de ses dérivés N-alkylés ou N,N-dialkylés, en faisant réagir de l'acide parahydroxymandélique ou un de ses sels avec de l'ammoniac ou une alkylamine ou une dialkylamine ou l'un de ses sels; mais ce procédé, avantageusement effectué en solution aqueuse, exige lorsqu'on travaille à la pression ambiante de longues périodes de chauffage:

30 heures à 103°C selon l'exemple 3, qui peuvent être sensiblement réduites en opérant sous pression; 4 heures à 135°C sous une pression de 1,55 kg/cm$^2$ selon l'exemple 4. Ces conditions associées à la manipulation d'amines peu condensées en carbone, volatiles et irritantes, rendent ce procédé coûteux et difficilement transposable à l'échelle industrielle.

La demanderesse a maintenant découvert qu'il est possible d'accéder facilement et avantageusement au diméthylamino-2 parahydroxyphényl-2 acétate de sodium cristallisé après chauffage de l'acide parahydroxymandélique monohydraté dans le diméthylformamide en présence d'une quantité catalytique d'un acide minéral fort.

L'invention a pour objet le diméthylamino-2 parahydroxyphényl-2 acétate de sodium cristallisé anhydre ou avec deux molécules d'eau.

L'invention a aussi pour objet un nouveau procédé pour obtenir avec de bons rendements le diméthylamino-2 parahydroxyphényl-2 acétate de sodium cristallisé anhydre ou avec deux molécules d'eau suivant lequel on chauffe de l'acide parahydroxymandélique dans le diméthylformamide en présence d'une quantité catalytique d'un acide minéral fort tel l'acide sulfurique concentré, puis on concentre à demi environ le milieu réactionnel.

A ce stade, on peut, soit filtrer la suspension obtenue et recueillir l'acide diméthylamino-2 parahydroxyphényl 2 acétique cristallisé pur qui est transformé ultérieurement en son sel de sodium cristallisé pur anhydre ou avec deux molécules d'eau, soit nmeutraliser cette suspension par addition de la quantité nécessaire d'hydroxyde de sodium en solution aqueuse et poursuivre la concentration sous vide jusqu'à siccité; dans ce cas on obtient le diméthylamino-2 parahydroxyphényl-2 acétate de sodium cristallisé avec deux molécules d'eau souillé de traces de sels de sodium de l'acide parahydroxymandélique de départ non transformé et de l'acide minéral fort utilisé comme catalyseur. L'acide minéral fort utilisé comme catalyseur est avantageusement l'acide sulfurique concentré.

L'acide diméthylamino-2 parahydroxyphényl-2 acétique est un produit cristallisé incolore, présentant un point de fusion instantané vers 260°C avec décomposition, un pKa de 2 et une très bonne solubilité dans l'eau. A l'état cristallisé, il est sous la forme d'ion dipolaire, comme le montrent les spectres infrarouges enregistrés à l'état solide. L'acide diméthylamino-2 parahydroxyphényl-2 acétique donne aussi un chlorhydrate cristallisé incolore, présentant un point de fusion instantané de 205 − 210°C (décomp.) et une excellente solubilité dans l'eau.

Le diméthylamino-2 parahydroxyphényl-2 acétate de sodium cristallisé anhydre ou avec deux molécules d'eau est obtenu par dissolution de l'acide diméthylamino-2 parahydroxyphényl-2 acétique dans la quantité théorique de soude N suivie d'une concentration à sec sous vide.

Par reprise du résidu cristallisé obtenu à l'acétone, essorage et séchage sous vide à 40°C à poids constant, on obtient le diméthylamino-2 parahydroxyphényl-2 acétate de sodium pur cristallisé avec deux molécules d'eau. Le sel anhydre cristallisé pur est préparé par séchage sous vide à poids constant à 100°C du sel pur cristallisé avec deux molécules d'eau.

L'invention a enfin pour objet l'application du diméthylamino-2 parahydroxyphényl-2 acétate de sodium à l'obtention du cyanure de parahydroxybenzyle.

Le cyanure de parahydroxybenzyle est un produit commercial, largement décrit dans la littérature. C'est un intermédiaire précieux pour accéder à divers produits industriels tels que la tyramine.

Selon l'invention, le cyanure de parahydroxybenzyle est avantageusement obtenu en faisant réagir du diméthylamino-2 parahydroxyphényl-2 acétate de sodium avec de la cyanhydrine de l'acétone (hydroxy-2 isobutyronitrile) et en distillant au fur et à mesure de sa formation l'acétone libérée; on dispose ainsi d'un moyen commode pour suivre l'avancement de la réaction. Avantageusement cette réaction est conduite à chaud dans du diméthylformamide. En fin de réaction, le cyanure de parahydroxybenzyle est isolé du milieu réactionnel selon des moyens connus en soi; avantageusement, on l'extrait avec un acétate d'alkyle en $C_2 − C_4$ (tel que l'acétate de butyle) du milieu réactionnel refroidi, on dilue à l'eau et amène le pH à 4 avec de l'acide acétique.

Par évaporation du solvant d'extraction, on obtient le cyanure de parahydroxybenzyle cristallisé que l'on peut ensuite, si nécessaire, purifier par rectification sous vide.

L'invention est illustrée sans être limitée par les exemples suivants:

## Exemple 1

On chauffe 150 mn à 135°C, une solution de 1 mole d'acide parahydromandélique monohydraté dans 13 moles de diméthylformamide et 0,1 mole d'acide sulfurique concentré d = 1.83. La suspension obtenue est ensuite concentrée sous vide à demi de son volume initial, puis elle est refroidie à la température ambiante. On essore le produit cristallisé formé, on le lave par empâtage avec 1 volume de méthanol puis on le sèchs sous vide à poids constant à 100°C.

On obtient ainsi 162 g d'acide diméthylamino-2 parahydroxyphényl-2 acétique cristallisé pur présentant un point de fusion instantané de 260°C avec décomposition, soit un rendement de 83% de la théorie. Cet acide est soluble dans l'eau, dans les bases diluées et dans les acides dilués il est insoluble dans le méthanol et l'acétone.

Microanalyse:
$C_{10}H_{13}NO_3$; P. M. = 195,21

calculés  C 61,53  H 6,71  N 7,17%;
trouvés   C 61,2   H 6,8   N 7,0%.

Infrarouge (pastille KBr): bandes larges vers 1400 et
1600 cm$^{-1}$: bandes caractéristiques de l'ion carboxylate.

Par concentration des eaux mères à demi, on isole un deuxième jet de 25 g d'acide diméthylamino-2 parahydroxyphényl-2 acétique cristallisé présentant un point de fusion instantané de 260°C avec décomposition, sans dépression en mélange avec de l'acide diméthylamino-2 parahydroxyphényl-2 acétique cristallisé pur. Le rendement global de la réaction s'établit ainsi à 95,8% de la théorie calculée par rapport à l'acide parahydroxymandélique mis en oeuvre.

Cet acide fournit un chlorhydrate qui cristallise du méthanol. Il est très soluble dans l'eau, peu soluble dans le méthanol et insoluble dans le diéthyle oxyde. Il présente un point de fusion peu net.

Microanalyse:
$C_{10}H_{13}NO_{13}$, HCl

calculés  C 51,83  H 6,09  N 6,05  Cl 15,31%;
trouvés   C 51,5   H 6,1   N 6,1  Cl 15,2%.

On dissout 39 g (o,2 mole) d'acide diméthylamino-2 parahydroxyphényl-2 acétique cristallisé pur dans 200 cm$^3$ de soude N. La solution limpide obtenue présentant un pH = 10,1 est ensuite concentrée sous vide à sec puis le résidu cristallisé est empâté à l'acétone, filtré et séché sous vide à 40°C à poids constant; on obtient ainsi 49,4 g (0,195 mole) de diméthylamino-2 parahydroxyphényl-2 acétate de sodium cristallisé pur avec deux molécules d'eau soit un rendement de 93% de la théorie.

Microanalyse:
$C_{10}H_{12}N\ NaO_3$, $2H_2O$; P. M: 253,24

calculés  C 47,42  H 6,37  N 5,53  $H_2O(1)$ 14,23%;
trouvés   C 47,2   H 6,5   N 5,4  $H_2O(1)$ 13,7%.

(1) déterminés par la méthode de K. Fischer.

Dosage potentiométrique:

— amine libre: 3,93 mèq/g soit 99,4% de la théorie;
— anion carboxylate: 3,92 mèq/g soit 99,2% de la théorie;
— fonction carboxylique: pKa = 2;
— fonction ammonium: pKa = 8,8;
— fonction phénol: pKa = 10,5.

## Exemple 2

Dans une solution de 0,22 mole (55,84 g) de diméthylamino-2 parahydroxyphényl-2 acétate de sodium cristallisé pur avec deux molécules d'eau dans 3,25 moles (250 cm$^3$) de diméthylformamide chauffée à 135°C, on introduit goutte à goutte en 4 heures 0,265 mole (22,47 g) d'hydroxy-2 isobutyronitrile à 98 ± 1% (cyanhydrine de l'acétone). L'acétone formée est distillée au fur et à mesure de l'avancement de la réaction. On poursuit le chauffage 2 h à 135°C après la fin d'introduction de la cyanhydrine de l'acétone, puis le milieu réactionnel refroidi à la température ambiante est dilué avec 1 l d'eau, amené à pH = 4 avec de l'acide acétique et extrait 3 fois avec 1 l d'acétate de butyle.

Les phases organiques réunies sont ensuite lavées 2 fois avec 1 l d'eau, séchées sur sulfate de sodium anhydre, filtrées puis concentrées à sec sous vide. On isole ainsi 26 g (0,195 mole) de cyanure de parahydroxybenzyle cristallisé présentant un point de fusion de 70°C (littérature: Beil., 10, 191, F = 69 − 71°C) soit un rendement de 89% de la théorie calculée par rapport au diméthylamino-2 parahydroxyphényl-2 acétate de sodium cristallisé pur avec deux molécules d'eau mis en oeuvre.

Il va de soi que la présente invention n'a été décrite qu'à titre purement explicatif et nullement limitatif et que toute modification utile pourra y être apportée sans sortir de son cadre.

## Revendications

1. A titre de produit industriel nouveau, le diméthylamino-2 parahydroxyphényl-2 acétate de sodium cristallisé.

2. Diméthylamino-2 parahydroxyphényl-2 acétate de sodium selon la revendication 1, caractérisé par le fait qu'il est cristallisé avec deux molécules d'eau.

3. Diméthyl-2 parahydroxyphényl-2 acétate de sodium selon la revendication 1, caracatérisé par le fait qu'il est cristallisé anhydre.

4. Procédé pour produire du diméthylamino-2 parahydroxyphényl-2 acétate de sodium cristallisé, caractérisé par le fait que l'on chauffe de l'acide parahydroxymandélique dans du diméthylformamide en présence d'une quantité catalytique d'un acide fort, réaction suivie d'une neutralisation à l'hydroxyde de sodium.

5. Procédé selon la revendication 4, caractérisé par le fait que l'on chauffe à une température comprise entre 110 et 140°C de l'acide parahydroxymandélique anhydre ou monohydraté dans diméthylformamide en présence d'une quantité catalytique d'acide sulfurique concentré.

6. Application du diméthylamino-2 parahydroxyphényl-2 acétate de sodium cristallisé selon l'une quelconque des revendications 1 à 3 à la préparation du cyanure de parahydroxybenzyle.

7. Application selon la revendication 6, caractérisée par le fait réagir du diméthylamino-2 parahydroxyphényl-2 acétate de sodium cristallisé avec de l'hydroxy-2 isobutyronitrile.

## Patentansprüche

1. Kristallisiertes Natrium-2-dimethylamino-2-parahydroxyphenyl-acetat als neues Industrieerzeugnis.

2. Natrium-2-dimethylamino-2-parahydroxyphenyl-acetat nach Anspruch 1, dadurch gekennzeichnet, daß es mit zwei Molekülen Wasser kristallisiert ist.

3. Natrium-2-dimethylamino-2-parahydroxyphenyl-acetat nach Anspruch 1, dadurch gekennzeichnet, daß es wasserfrei kristallisiert ist.

4. Verfahren zur Herstellung von kristallisiertem Natrium-2-dimethylamino-2-parahydroxyphenyl-acetat, dadurch gekennzeichnet, daß man Parahydroxymandelsäure in Dimethylformamid in Gegenwart einer katalytischen Menge einer starken Säure erhitzt und anschließend mit Natriumhydroxid neutralisiert.

5. Verfahren nach Anspruch 4, dadurch gekennzeichnet, daß man Parahydroxymandelsäure, wasserfrei oder als Monohydrat, in Dimethylformamid in Gegenwart einer katalytischen Menge konzentrierter Schwefelsäure bei einer Temperatur im Bereich von 110 bis 140°C erhitzt.

6. Verwendung von kristallisiertem Natrium-2-dimethylamino-2-parahydroxyphenyl-acetat nach einem der Ansprüche 1 bis 3 zur Herstellung von Parahydroxybenzylcyanid.

7. Verwendung nach Anspruch 6, dadurch gekennzeichnet, daß man kristallisiertes Natrium-2-dimethylamino-2-parahydroxyphenyl-acetat mit 2-Hydroxy-isobutyroniril reagieren läßt.

## Claims

1. As a novel industrial product, crystallized sodium 2-dimethylamino-2-parahydroxyphenyl acetate.

2. Sodium 2-dimethylamino-2-parahydroxyphenyl acetate as in claim 1, characterized in that it is crystallized with two molecules of water.

3. Sodium 2-dimethylamino-2-parahydroxyphenyl acetate as in claim 1, characterized in that it is crystallized anhydrous.

4. A process for producing crystallized sodium 2-dimethylamino-2-parahydroxyphenyl acetate, characterized by heating parahydroxymandelic acid in dimethylformamide in the presence of a catalytic quantity of a strong acid, this reaction being followed by sodium hydroxide neutralization.

5. A process as in claim 4, characterized by heating anhydrous or monohydrated parahydroxymandelic acid at a temperature of between 110 and 140°C in dimethylformamide in the presence of a

0 082 762

catalytic quantity of concentrated sulfuric acid.

6. The application of crystallized sodium 2-dimethylamino-2-parahydroxyphenyl acetate as in any one of claims 1 to 3, for the preparation of parahydroxybenzyl cyanide.

7. An application as in claim 6, characterized by reacting crystallized sodium 2-dimethylamino-2-parahydroxyphenyl acetate with 2-hydroxy-isobutyronitrile.

5